(19) Europäisches Patentamt
European Patent Office
Office européen des brevets

(11) Veröffentlichungsnummer: **0 518 013 A2**

# (12) EUROPÄISCHE PATENTANMELDUNG

(21) Anmeldenummer: **92105667.7**

(51) Int. Cl.$^5$: **C07C 213/06**

(22) Anmeldetag: **02.04.92**

(30) Priorität: **14.06.91 DE 4119576**

(43) Veröffentlichungstag der Anmeldung:
**16.12.92 Patentblatt 92/51**

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**W-4370 Marl 1(DE)**

(72) Erfinder: **Steffen, Klaus-Dieter, Dr.**
**Röckelstrasse 36**
**W-5202 Hennef 1(DE)**

## (54) Verfahren zur Herstellung von 1-Alkoxy-2-dialkylaminoethanen.

(57) 1-Alkoxy-2-dialkylamino-ethane werden aus Dialkylamino-ethanol durch Umsetzung mit Alkalialkoholat und danach mit Alkylhalogeniden erhalten, wobei der aus dem Alkoholat gebildete Alkohol vollständig durch Destillation unter Zusatz des Reaktionsproduktes oder eines Lösungsmittels, das mit dem Alkohol ein Azeotrop bildet, erfolgt und die Reindestillation in Gegenwart von Alkali-Alkoholat in mindestens äquivalenten Mengen zum Dialkylamino-ethanol erfolgt.
Hohe Ausbeute und Reinheit wird durch Einsatz der Alkali-$\beta$-dialkylamino-ethanolats in die Alkylierung erreicht.

EP 0 518 013 A2

Gegenstand der Erfindung ist ein Verfahren zur Herstellung von 1-Alkoxy-2-dialkylamino-ethanen der allgemeinen Formel (III) aus $\beta$-Dialkylamino-ethanol (I) und Alkylhalogeniden $R_2$ - X (II)

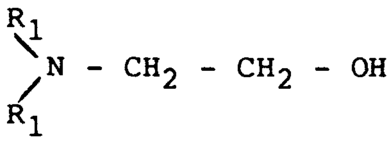

(I)

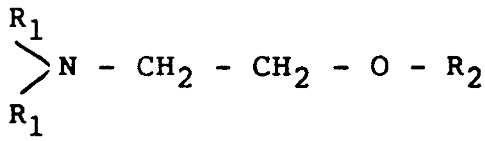

(III)

$R_1$ = Alkyl $C_1$ - $C_{10}$, vorzugsweise $C_1$ bis $C_3$

$R_2$ = Alkyl $C_1$ - $C_{10}$, gleich oder verschieden zu $R_1$, vorzugsweise $C_1$ bis $C_3$

X = Cl, Br, J,

dadurch gekennzeichnet, daß man

a) $\beta$-Dialkylamino-ethanol (I) mit Natrium- oder Kaliumalkoholaten einfacher Alkohole, bevorzugt Methanol, umsetzt unter restloser Entfernung des einfachen Alkohols,

b) den Dialkylamino-ethanol (I) im Überschuß von 0,1 bis 30, bevorzugt 5 bis 20 Mol-%, bezogen auf Alkalialkoholat, anwendet,

c) die restlose Entfernung des einfachen Alkohols durch Abdestillation mit inerten Lösemitteln wie aliphatischen oder aromatischen Kohlenwasserstoffen, bevorzugt aber mit dem herzustellenden 1-Alkoxy-2-dialkylamino-ethan selbst vornimmt,

d) die Umsetzung des nach a) bis c) entstandenen Alkali-$\beta$-dialkylamino-ethanolates mit Alkylhalogeniden, bevorzugt mit Alkylchloriden, bei erhöhten Temperaturen bei Atmosphärendruck oder erhöhtem Druck durchführt,

e) die Reindestillation des Zielproduktes 1-Alkoxy-2-dialkylamino-ethan unter vorheriger Zugabe von Na-/K-Alkoholat vornimmt,

f) diese Na-/K-Alkoholate nach e) in Mengen zufügt, die dem restlichen Dialkylamino-ethanol mindestens stöchiometrisch äquivalent sind, bevorzugt aber im Überschuß von bis zu 15 Mol-% eingesetzt werden,

g) dieses im Sumpf der Reindestillation verbleibende Alkali-$\beta$-dialkyl-amino-ethanolat in die Alkylierungs-Reaktion der Folgesätze überführt.

1-Alkoxy-2-dialkylamino-ethane sind lange bekannt. Die Herstellung erfolgte durch Umsetzung von 1-Dimethylaminoethylchlorid-2 mit Natriummethylat. Die dort ohne Ausbeute-Angaben getroffenen Reaktions- und Aufarbeitungsbedingungen sind schwierig und ergeben geringe Reinheit.

Nach dem U.S.-Patent 4,588,843 erfolgt die Alkali-Alkoholat-Bildung von $\beta$-Dialkylamino-alkanol mit NaOH/KOH und die anschließende Umsetzung mit Alkylhalogeniden. Die Aufarbeitung aus einer wäßrigen Phase durch Extraktion mit Ether ist umständlich und ergibt nur 37% Ausbeute.

Aufgabe für die Herstellung von 1-Alkoxy-2-dialkylaminoethan muß also sein, ein technisch einfaches Verfahren zu finden, nach dem das Zielprodukt in hohen Ausbeuten und Reinheiten erhalten wird.

Nach dieser Erfindung geht das Verfahren von $\beta$-Dialkylamino-ethanol aus, ein Produkt, das großtechnisch einfach aus Ethylenoxid und Dialkylaminen herzustellen ist.

Dieser $\beta$-Dialkylamino-alkohol muß quantitativ in sein Alkali-alkoholat überführt werden, was erfindungsgemäß mit Alkali-alkoholaten durchgeführt wird. Bevorzugt sind die Natrium- und Kaliumalkoholate kurzkettiger Alkohole mit 1 bis 3 C-Atomen, besonders Natriummethanolat. Bevorzugt sind weiter Alkoholat als Lösung im jeweiligen Alkohol oder auch als isoliertes Salz.

Die Alkalialkoholate werden im Unterschuß zum $\beta$-Dialkylamino-ethanol(I), maximal in stöchiometrisch äquivalenten Mengen eingesetzt. Dadurch erfolgt die Alkalisalz-Bildung des $\beta$-Dialkylaminoethanols schneller und einfacher, und es bleibt kein Alkali-alkoholat übrig, das mit dem Alkylhalogenid sofort leichtsiedenden Alkylether bilden würde.

Der molare Überschuß an (I) beträgt 0,1 bis 30%, bevorzugt 5 bis 20%, und wird später bei der Reindestillation als Alkalisalz gebunden, wie noch auszuführen sein wird.

Die Schwierigkeit besteht hierbei in der restlosen Entfernung des kurzkettigen Alkohols, d.h. in der quantitativen Überführung in das Natrium-$\beta$-dialkylamino-ethanolat. Dies wird dadurch erreicht, daß man das Methanol mit einem inerten Lösemittel destillativ rausschleppt, wobei dieses Lösemittel mit Methanol auch ein Azeotrop bilden kann, z.B. Toluol, Xylol, Alkane mit 5 bis 8 C-Atomen wie Hexan, Heptan, Cyclohexan, t-Butylmethylether. Wegen der Schwierigkeiten der Rückgewinnung dieses Lösemittels, d.h. der Abtrennung des Methanols, wird bevorzugt das Zielprodukt, d.h. das jeweilige 1-Alkoxy-2-dialkylamino-ethan eingesetzt, insbesondere nach dem zweiten oder dritten Ansatz. Die Verwendung dieses noch nicht so reinen

Endproduktes hat den großen Vorteil, daß kein Fremdlösemittel anschließend aufgearbeitet, d.h. von Methanol getrennt werden muß und die Reinheit des Endprodukts nicht von Spuren des fremden Lösemittels verunreinigt werden kann.

Weiterhin ist die Löslichkeit des Alkali-β-dialkylamino-ethanolates hierin besser als in einem artfremden Lösemittel.

Wenn die Suspension des Alkali-β-dialkylaminoethanols vorliegt, erfolgt anschließend die Umsetzung mit Alkylhalogeniden, z.B. Ethylchlorid, Methylbromid u.a., wobei hier die Alkylchloride bevorzugt werden, da mit ihnen höhere Ausbeuten zu erzielen sind als mit den Alkylbromiden.

Die Alkylhalogenide werden in stöchiometrischen Mengen oder geringem Überschuß bis zu 15 Mol-% eingesetzt. Überschüssiges, nicht abreagiertes Alkylhalogenid kann durch Druckentspannung in das Alkalisalz von (I) des Folgeansatzes rückgeführt werden. Die Umsetzung erfolgt bei Temperaturen von 30 bis 160° C, bevorzugt zwischen 50 und 120° C, bei Normaldruck oder auch unter Drücken bis 20 bar. Das Alkylhalogenid wird hierbei bei der Reaktionstemperatur in einem Zeitraum von 50 bis 80 Minuten zudosiert, z.T. mit einer Dosierpumpe gegen den Druck der Reaktionslösung. Die Reaktion ist leicht exotherm und die Reaktionszeit richtet sich daher nach der Größe der Ansätze und nach der Möglichkeit der Wärmeabführung. Die Nachreaktionszeit beträgt nochmals zwei bis sechs Stunden.

Nach Reaktionsende ist das Alkalihalogenid, z.B. NaCl, abzufiltrieren oder der gesamte flüchtige Anteil vom Salz abzudestillieren.

Als letzter Aufarbeitungsschritt folgt die Reindestillation. Leider bildet das Ausgangsprodukt β-Dialkylamino-ethanol mit vielen Lösungsmitteln wie Toluol, Cyclohexan, die jeweiligen Azeotrope, die Lösungsmittelgehalte von nur wenigen Prozenten aufweisen. Auch von dem Zielprodukt, z.B. 1-Dimethylamino-2-ethoxy-ethan ist β-Dimethylamino-ethanol nur unter großem Destillationsaufwand über Kolonnen mit einer theoretischen Stufenzahl mehr als 50 abzudestillieren. Daher ist es zweckmäßig, den Dialkylamino-ethanol im rohen Zielprodukt so weit wie möglich zu senken. Dies ist möglich, wenn man entsprechend dem geringen Gehalt an β-Dialkylamino-ethanol Alkali-alkoholat zusetzt und so den Dialkylamino-alkohol als Alkoholat-Salz im Destillationssumpf bindet. Bevorzugt setzt man einen 5 bis 15 Mol-%igen Überschuß an Alkalialkoholat ein. Bei der Reindestillation wird daher zuerst der einfache Alkohol als Vorlauf abdestilliert, bevor als Hauptlauf das Zielprodukt überdestilliert. Diese Reindestillation kann bei Normaldruck oder unter leichtem Vakuum erfolgen und erfordert keinen großen Trennaufwand.

Auf diese Art und Weise werden Reinheiten an 1-Alkoxy-dialkylamino-2-ethanen von 99,9% erzielt.

Im Destillationssumpf verbleibt das Alkalisalz des Dialkylamino-ethanols neben etwas Alkalialkoholat suspendiert im Zielprodukt, das in den Synthesereaktor zurückgeführt wird. Dadurch treten keine Verluste an Ausgangsprodukt auf. Die Ausbeuten an 1-Alkoxy-2-dialkylamino-ethan liegen aus diesem Grunde auch sehr hoch bei 95% der Theorie.

**Beispiel 1 (zum Vergleich)**
**1-Ethoxy-2-dimethylamino-ethan (EDE)**

In einen Glasautoklaven, der mit Rührer, Temperaturmessung, druckfestem Destillationsteil und Alkylhalogenid-Dosierpumpe ausgerüstet ist, werden 359 g 30 %ige Natriummethanolat-Lösung (2,0 Mol) eingefüllt und das Methanol bis zu einer Sumpftemperatur von 130° C abdestilliert. Dann werden 214 g β-Dimethylamino-ethanol DMAE (2,4 Mol) und 700 ml Cyclohexan zugegeben und das gesamte Methanol abdestilliert bis zu einer Sumpftemperatur von 85° C.

Nach dem Ankühlen auf 25° C sind 22 g Ethylbromid (2,04 Mol) in 1 St. einzupumpen und die Reaktion bei 65° C in 4 bis 5 St. zu Ende zu bringen. Nach dem Abkühlen auf 20° C wird NaBr abfiltriert, mit Cyclohexan nachgewaschen und getrocknet: 219 g (106 % d.Th. durch quater. Ammoniumsalze). Das Filtrat wird an einer 1 m langen Laborkolonne rektifiziert. Zuerst destilliert Cyclohexan ab, dann folgt der Hauptlauf und ein Dimethylamino-ethanol-reicher Nachlauf (Kp.: 95bis98° C bei 500 mbar).

| | |
|---|---|
| Hauptlauf: | 196,7 g mit 94,1% EDE, 5,4% DMAE |
| Nachlauf: | 30,3 g mit 3,3% EDE, 89,3% DMAE |
| Ausbeute: | 186,1 g EDE (79,5% d.Th.) |

**DMAE-Rückgewinnung:**

als Azeotrop mit Cyclohexan, in der Haupt- und Nachfraktion destillieren insgesamt 60 g DMAE (0,67 Mol) ab (ermittelt aus Auswaage und G.C.-Gehalt), was dem Überschuß (35,6 g) und nicht abreagiertem Produkt nicht ganz entspricht.

**Beispiel 2 (zum Vergleich)**
**1-Ethoxy-2-dimethylamino-ethan (EDE)**

In dem Autoklaven (wie in Beispiel 1 beschrieben) werden 359 g 30 Gew.-%ige methanolische Natriummethanolat-Lösung (2,0 Mol) zur Trockne eingeengt und dann 172 g β-Dimethylamino-ethanol sowie aus Vorversuchen 800 g Cyclohexan, das noch 42 g DMAE gelöst enthält (zusammen 214 g DMAE, 2,4 Mol) eingefüllt. Das gesamte Methanol wird als Azeotop abdestilliert, dann der Autoklave verschlossen und bei ca. 100 °C sind 142 g Ethylchlorid (2,2 Mol) bei einem Druck von 2 bis 3 bar in 1,5 St. einzupumpen. Die Nachreaktionszeit beträgt 3 Std. Nach dem Abkühlen wird das NaCl abfiltriert, gewaschen und getrocknet (110g, 94,1% d.Th.).
Die gesamten Filtrate werden über eine 1 m lange Laborkolonne bei einem Vakuum von 500 mbar und unter Rückfluß rektifiziert. Nach Abnahme von Cyclohexan destillieren ein EDE-Hauptlauf und ein kleiner DMAE-Nachlauf.

Hauptlauf: 222 g mit 94,7% EDE, 4,9% DMAE
Nachlauf: 15,5 g mit 64,4% EDE, 35 % DMAE
Ausbeute: 220,2 g EDE (94,0% d.Th.).

Der DMAE-Überschuß (0,4 Mol) verteilt sich auf die beiden Destillationsfraktionen (16,3g) und im Cyclohexanvorlauf.

**Beispiel 3 (Erfindung)**
**1-Ethoxy-2-dimethylamino-ethan (EDE)**

In der vorn beschriebenen Apparatur werden 361 g 29,9 Gew.-%iges methanolisches NaOCH (2,0 Mol) zur Trockne eingedampft und 188 g DMAE mit 800 EDE, das noch 8 g DMAE enthält (zusammen DMAE: 196 g, 2,2 Mol) eingefüllt.
Das gesamte Methanol wird abdestilliert. Bei 100 °C werden in die verschlossene Apparatur innerhalb von 1,5 Std. 142 g Ethylchlorid (2,2 Mol) eingepumpt bei einem Druck von ca. 1,5 bar. Nach einer Nachreaktionszeit von 3 Std. bei 100 °C ist die Reaktion beendet.
Das gesamte EDE wird bei 500 mbar Vakuum vom NaCl abdestilliert.
(NaCl: 117g, 100% d.Th.).

Es fallen 1106 g Destillat an mit einem DMAE-Gehalt von 2,9% (entsprechend 0,36 Mol). Zur Bindung als Na-Salz werden 72 g 30 Gew.-%ige methanolisches NaOCH,-Lösung (0,40 Mol) zugesetzt und die gesamte Mischung über eine Kolonne aufdestilliert. Nach Abnahme des gesamten Methanols und eines kleinen Zwischenlaufes destilliert sehr reines EDE bei 120 °C über. Im Destillat-Sumpf verbleibt das Na-Salz von DMAE mit etwas EDE, das dem Folgesatz bei der Na-DMAE-Bildung zugeschlagen wird.

Ausbeute: 1015 g (abzgl. 800 g Lösemittel) 215 g EDE (91,9% d.Th. einschließlich Sumpf 95%)
G.C.-Reinheit: 99,9%.

Ausbeute nach vier Ansätzen mit Rückführung von EDE und Na-DMAE ergeben 96% Ausbeute.

**Beispiel 4 (Erfindung)**
**1-Ethoxy-2-dimethylamino-ethan (EDE)**

Der Destillationssumpf aus Beispiel 3, der 0,36 Mol Na-Salz des DMAE und 0,04 Mol NaOCH enthält, wird mit frische 1,96 Mol NaOCH-Lösung versetzt und das Methanol abdestilliert. Anschließend werden 1,84 Mol DMAE (164 g) und 800 g EDE zugefügt und unter Abdestillieren des gesamten Methanols das Na-DMAE gebildet.
Die weitere Reaktionsabfolge verläuft wie im Beispiel 3 beschrieben.
Die Ausbeute an EDE beträgt 222 g (95% d.Th.)
Nach vier Ansätzen mit Rückführung von EDE und Na-DMAE ergibt sich eine Ausbeute von 97% und eine Reinheit von 99,8%.

**Beispiel 5 (Erfindung)**
**1-Ethoxy-2-diethylamino-ethan (EDEE)**

In dem in Beispiel 1 beschriebenen Autoklaven werden 359 g 30 Gew.-%ige NaOCH3-Lösung (2,0 Mol) zur Trockne eingeengt (130 °C), 281 g 2-Diethylaminoethanol (DEAE, 2,4 Mol) und 1000 ml t-Butylmethylether eingefüllt und das Mol) und 1000 ml t-Butyl-methylether eingefüllt und das gesamte Methanol

azeotrop abdestilliert. Bei einer Temperatur von 100 °C werden 142 g Ethylchlorid (2,2 Mol) innerhalb von 1,5 Std. zugepumpt und die Reaktion anschließend noch 3 Std. zu Ende geführt (Druck: 1 bis 3,5 bar). Nach dem Abkühlen wird das NaCl abfiltriert, mit t-Butylmethylether gewaschen und getrocknet (114 g, 97,5 % d.Th.)
Die gesammelten Filtrate werden über eine Kolonne aufdestilliert; nach Abnahme des Lösemittels destilliert EDEE mit 5% DEAE als Hauptfraktion über. Im Destillationssumpf verbleibt eine kleine DEAE-reiche Fraktion. Die Hauptfraktion wird mit der 1,1-fachen äquivalenten Menge an NaOCH3-Lösung - bezogen auf restliches DEAE - versetzt und nochmals der fraktionierten Destillation unterworfen. Nach Abdestillation des Methanol-Vorlaufes destilliert sehr reines EDEE bei 710 °C/40 mbar über. Der aus dieser Reindestillation verbleibende Sumpf, der das Na-Salz aus DEAE enthält, wird für Folgeansätze wieder eingesetzt, in denen dann EDEE als Lösungsmittel (statt t-Butylmethylether) verwendet wird; bei mehrfach wiederholtem Wiedereinsatz des Destillations-Sumpfes steigen die Ausbeuten über 90% an.
Ausbeute:240,6 g (82,9%, bezogen auf Na-Methylat; einschließlich Sumpf 95%.
Nach viermaliger Rückführung werden Ausbeuten von 95% und Reinheiten über 99,5% erhalten.

Beispiele 6 bis 9

Beispiel 6: 1-Propoxy-2- dimethylamino-ethan (PDME)
Beispiel 7: 1-Butoxy -2- dimethylamino-ethan (BDME)
Beispiel 8: 1-Propoxy-2- diethylamino -ethan (PDEE)
Beispiel 9: 1-Butoxy -2- diethylamino -ethan (BDEE)

Entsprechend den Vorschriften des Beispiels 5 werden in den Lösungsmitteln Cyclohexan oder t-Butylmethylether sowohl β-Dimethyl- als auch β-Diethylamino-ethanol mit n-Propylchlorid und n-Butylchlorid umgesetzt zu den entsprechenden Amino-alkylethern.

Die Reaktionstemperaturen betragen unter einem Druck von 1 bis 3 bar 100 bis 115 °C im Autoklaven.

Die maximalen Ausbeuten und Reinheiten werden erst nach zwei bis vier Ansatz-Cyclen bei Einsatz des Zielproduktes als Lösemittel und Rückführung des Na-Dialkylamino-ethanolates aus dem Destillationssumpf erhalten.

| Beispiel | Einsatzstoffe | | | | | Zeiten [h] | | Ausb. [% d.Th.] bzg. a. verbrauchten Amino-alkohol | G.C.-Reinheit [%] |
|---|---|---|---|---|---|---|---|---|---|
| | NM 30 | Amino-ethanol | | Alkylchlorid | | Dosieren | Nachreakt. | | |
| | [g/Mol] | Typ | [g/Mol] | Typ | [g/Mol] | | | | |
| 6 | 359/2,0 | DMAE | 214/2,4 | n-Pr.-Cl | 173/2,2 | 1,25 | 6 | 95 | 99.3 |
| 7 | 359/2,0 | DMAE | 214/2,4 | n-But.-Cl | 203/2,2 | 1,5 | 6 | 95 | 99.6 |
| 8 | 359/2,0 | DEAE | 281/2,4 | n-Pr.-Cl | 173/2,2 | 1,5 | 6 | 95 | 98.5 |
| 9 | 359/2,0 | DEAE | 281/2,4 | n-But.-Cl | 204/2,2 | 1,5 | 6 | 94 | 98.5 |

nach jeweils 4 Ansätzen mit Rückführung

## Patentansprüche

1. Verfahren zur Herstellung von 1-Alkoxy-2-dialkylamino-ethanen der allgmeinen Formel (III) aus $\beta$-Dialkylamino-ethanol (1) und Alkylhalogeniden $R_2$ - X (II)

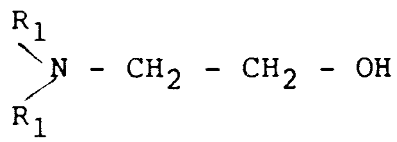

(I)

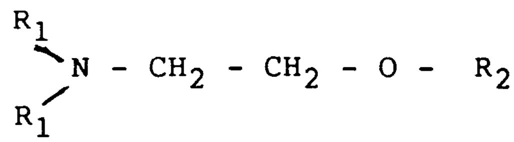

(III)

$R_1$ = Alkyl $C_1$ - $C_{10}$
$R_2$ = Alkyl $C_1$ - $c_{10}$
X = Cl, Br, J,
dadurch gekennzeichnet, daß man

a) $\beta$-Dialkylamino-ethanol (I) mit Natrium oder Kaliumalkoholaten einfacher Alkohole, bevorzugt Methanol, umsetzt unter restloser Entfernung des einfachen Alkohols,
b) den Dialkylamino-ethanol (I) im Überschuß von 0,1 bis 30, bevorzugt 5 bis 20 Mol-%, bezogen auf Alkalialkoholat anwendet,
c) die restlose Entfernung des einfachen Alkohols durch Abdestillation mit inerten Lösemitteln wie aliphatischen oder aromatischen Kohlenwasserstoffen, bevorzugt aber mit dem herzustellenden 1-Alkoxy-2-dialkylamino-ethan selbst vornimmt,
d) die Umsetzung des nach a) bis c) entstandenen Alkali-$\beta$-dialkylamino-ethanolates mit Alkylhalogeniden, bei erhöhten Temperaturen bei Atmosphärendruck oder erhöhtem Druck durchführt,
e) die Reindestillation des Zielproduktes 1-Alkoxy-2-dialkylamino-ethan unter vorheriger Zugabe von Na-/K-Alkoholat vornimmt.